(19) Europäisches Patentamt
European Patent Office
Office européen des brevets

(11) Veröffentlichungsnummer: **0 321 504 B1**

(12) # EUROPÄISCHE PATENTSCHRIFT

(45) Veröffentlichungstag der Patentschrift: **08.01.92 Patentblatt 92/02**

(51) Int. Cl.[5]: **C07C 69/712,** C07C 69/92, C07D 239/26, C07D 239/34, C09K 19/20

(21) Anmeldenummer: **87907752.7**

(22) Anmeldetag: **02.11.87**

(86) Internationale Anmeldenummer: **PCT/EP87/00653**

(87) Internationale Veröffentlichungsnummer: **WO 88/03525 19.05.88 Gazette 88/11**

(54) **CHIRALE ARYLOXYPROPIONSÄUREESTER UND IHRE VERWENDUNG ALS DOTIERSTOFF IN FLÜSSIGKRISTALL-PHASEN.**

(30) Priorität: **08.11.86 DE 3638119**

(43) Veröffentlichungstag der Anmeldung: **28.06.89 Patentblatt 89/26**

(45) Bekanntmachung des Hinweises auf die Patenterteilung: **08.01.92 Patentblatt 92/02**

(84) Benannte Vertragsstaaten: **AT BE DE FR GB IT NL SE**

(56) Entgegenhaltungen:
**EP-A- 0 175 591**
**WO-A-87/05012**

(73) Patentinhaber: **HOECHST AKTIENGESELLSCHAFT**
**Postfach 80 03 20**
**W-6230 Frankfurt am Main 80 (DE)**

(72) Erfinder: **WINGEN, Rainer**
**Rotkäppchenweg 10**
**W-6234 Hattersheim am Main (DE)**
Erfinder: **DÜBAL, Hans-Rolf**
**Kirschgartenstrasse 10**
**W-6238 Hofheim am Taunus (DE)**
Erfinder: **HEMMERLING, Wolfgang**
**Billtalstrasse 32**
**W-6231 Sulzbach (DE)**
Erfinder: **MÜLLER, Ingrid**
**Am Pfingstbrunnen 1**
**W-6238 Hofheim am Taunus (DE)**
Erfinder: **OHLENDORF, Dieter**
**Am Kühlen Grund 4**
**W-6237 Liederbach (DE)**

**EP 0 321 504 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Flüssigkristalle haben insbesondere im letzten Jahrzehnt Eingang in verschiedenste technische Gebiete gefunden, in denen elektrooptische und Anzeigevorrichtungs-Eigenschaften verlangt werden (z.B. in Uhren-, Taschenrechner- und Schreibmaschinenanzeigen). Diese Anzeigevorrichtungen machen Gebrauch von den dielektrischen Ausrichtungseffekten in den nematischen, cholesterischen und/oder smektischen Phasen der flüssigkristallinen Verbindungen, wobei -verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu langsam. Dieser Nachteil macht sich insbesondere dann bemerkbar, wenn - was bei größeren Anzeigeelementflächen zwangsläufig der Fall ist - eine große Anzahl von Bildpunkten angesteuert werden muß, wodurch die Produktionskosten großflächiger Geräte wie Videogeräte, Oszillographen oder Fernseh-, Radar-, EDV- oder Schreibautomaten-Bildschirme zu hoch wären.

Neben den nematischen und cholesterischen Flüssigkristallen haben seit einigen wenigen Jahren in zunehmenden Maße auch geneigt("tilted")-smektische Flüssigkristall-Phasen an Bedeutung gewonnen. Wenn solchen geneigt-smektischen Phasen, insbesondere smektisch C ($S_c$ oder SmC)Phasen, geeignete Dotierstoffe zugesetzt werden, die eine sogenannte spontane Polarisation ($P_s$) zeigen oder in der Flüssigkristall-Phase induzieren, so können die Phasen in ferroelektrische Flüssigkristall-Phasen umgewandelt werden (Benennung von $P_S$ in $nC \cdot cm^{-2}$), siehe dazu beispielsweise Lagerwall et al. im Aufsatz "Ferroelectric Liquid Crystals for Displays" (Ferroelektrische Flüssigkristalle für Anzeigeelemente), SID Symposium, October Meeting, 1985, San Diego (USA). Diese FLC-Phasen weisen - verglichen mit den üblichen TN ("twisted nematic")-Zellen - um etwa den Faktor 1000 schnellere Schaltzeiten auf, so daß sie, auch aufgrund anderer positiver Eigenschaften wie einer bistabilen Schaltmöglichkeit, gute potentielle Kandidaten für die oben angeführten Anwendungsgebiete (z.B. über eine Matrixansteuerung) sind.

In der WO-A 86/02937 werden als Komponenten ferroelektrischer Flüssigkristall-Mischungen u.a. Verbindungen der nachstehenden Formel

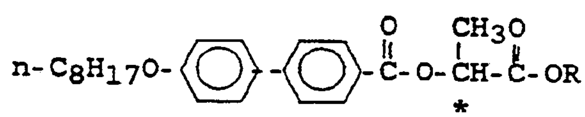

beschrieben, bei denen es sich um an der Hydroxylgruppe durch Arylcarbonsäuren veresterte 2-Hydroxypropionsäureester handelt, und R = Methyl, Ethyl, Propyl und n-Butyl bedeuten kann.
Die spontane Polarisation $P_S$ für diese Verbindungen (bestimmt in einer 10 mol-%-Mischung mit racemischem CE 8) wird bei einer Temperatur 10° unterhalb des $S_c^*$-$S_A$-Übergangs auf 72,6 $nC \cdot cm^{-2}$ (extrapoliert auf Reinsubstanz) angegeben; Schaltzeiten oder andere anwendungsrelevante Parameter werden nicht mitgeteilt.

In der EP-A 0175591 werden als Bestandteile von Flüssigkristall-Mischungen u.a. Verbindungen der nachstehenden Formel

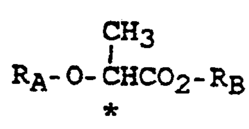

beschrieben, bei denen die Symbole folgende Bedeutung haben:
$R_A$: ein linearer, verzweigter oder cyclischer gesättigter oder ungesättigter Kohlenwasserstoffrest von 1 bis 20 C-Atomen;
$R_B$:

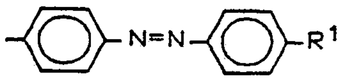 , 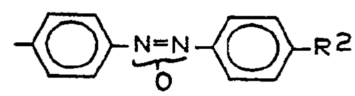 ,

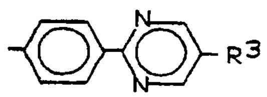,
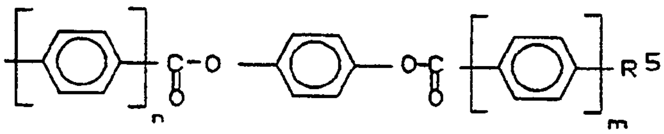,

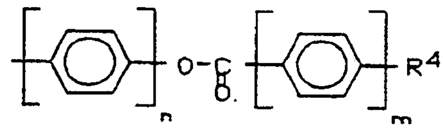,

wobei $R^1$ bis $R^5$ Alkyl- oder Alkyloxy-Gruppen mit 1 bis 20 C-Atomen und n bzw. m 1 oder 2 bedeuten sollen. Diese Verbindungen werden beschrieben als vorteilhaft zur Erzielung bistabilen Verhaltens in ferroelektrischen Flüssigkristall-Mischungen; optisch aktive C-Atome in $-R_8$ werden nicht genannt.

In der nicht-vorveröffentlichten WO-A 87/05012 werden u. a. Aryloxypropionsäureester beschrieben, die sich aber dadurch von den erfindungsgemäßen Verbindungen unterscheiden, daß der Alkylrest bzw. Alkoxyrest am rechten Molekülende in der WO-A $C_1$- bis $C_{12}$-Alkyl bzw. $C_1$- bis $C_{12}$-Alkyloxy bedeutet. Es wird zwar ausgeführt (Seite 2, Zeilen 31 bis 34), daß diese Alkylgruppe geradkettig, verzweigt oder "chiral" sein könne, bei Berücksichtigung konkreter Verbindungen aus Tabelle 3 (S. 4) und der Definition für "$R_2$" (Seite 5, Zeilen 2 bis 4) fallen jedoch nur solche asymmetrischen C-Atome unter die Definition von $R_2$, die über eine Carboxylgruppe mit dem Restmolekül verknüpft sind.

Gegenstand der Erfindung sind nun Verbindungen, die bei hohen Werten für die eigene oder in Flüssigkristall-Phasen induzierte spontane Polarisation $P_5$ Strukturelemente aufweisen, die sie auch mit anderen Komponenten in Flüssigkristall-Systemen, insbesondere mit Ester-Verbindungen mit $S_c$-Phase oder Phenylpyrimidinverbindungen mit $S_c$-Phase "verträglich", d.h. mischbar machen, da u.a. der mesogene Teil der Moleküle oftmals für eine gute "Verträglichkeit" mit den anderen Mischungskomponenten in Flüssigkristall-Systemen verantwortlich ist. Dabei müssen diese Verbindungen selbst nicht unbedingt flüssigkristallin sein, insbesondere nicht unbedingt eine SmC-Phase aufweisen.

Gegenstand der Erfindung sind insbesondere auch die neuen, in Tabelle zusammengefaßten Verbindungen.

Die erfindungsgemäßen chiralen Aryloxypropionsäureester sind Verbindungen der Formel (I); sie sind dadurch gekennzeichnet, daß in der allgemeinen Formel (I)

$$R^1\text{-}A\text{-}O\text{-}\underset{*}{\overset{CH_3}{\overset{|}{C}H}}\text{-}\overset{O}{\overset{\|}{C}}\text{-}O\text{-}B\text{-}R^2 \qquad (I)$$

$R^1$, A, B und $R^2$ bedeuten:

$R^1$ einen geradkettigen Alkyl- oder Alkyloxyrest mit 1 - 16 C-Atomen oder einen verzweigten Alkyl- oder Alkyloxyrest mit 4 - 16 C-Atomen, wobei eine oder zwei nicht - benachbarte $CH_2$-Gruppen durch ein S und/oder O ersetzt sein können;

A einen, zwei oder drei aromatische oder heteroaromatische Ringe, die direkt oder durch eine bzw. zwei COO-Gruppe(n) miteinander verknüpft sind;

B eine chemische Bindung oder einen oder zwei aromatische oder heteroaromatische Ringe, die direkt miteinander verknüpft sind;

$R^2$ a) wenn B eine chemische Bindung ist, einen Alkylrest mit 2 - 10 C-Atomen, der ein asymmetrisches C-Atom enthält, das mit $CH_3$, Halogen oder einer $COOC_2H_5$-Gruppe substituiert ist, oder einen Alkylrest mit 3 - 10 C-Atomen, der zwei benachbarte asymmetrische C-Atome enthält, von denen eines mit Halogen und das andere mit einer $CH_3$-Gruppe substituiert ist, oder die gemeinsam mit einem O einen Oxiranring bilden; oder

b) wenn B einen oder zwei aromatische oder heteroaromatische Ringe bedeutet, die direkt miteinander verknüpft sind, einen Alkyloxyrest mit derselben Anzahl von C-Atomen und demselben Aufbau der Alkylgruppe wie unter a).

Bei A soll unter dem aromatischen Ring vorzugsweise eine 1,4-Phenylen-Gruppierung, unter dem heteroaromatischen Ring bevorzugt ein Pyrimidin-2,5-diyl verstanden werden. Ebenfalls bevorzugt sind Verbindungen, bei denen zwei 1,4-Phenylen-Kerne über eine Carbonyloxy-Gruppe oder drei 1,4-Phenylen-Kerne über

zwei Carbonyloxy-Gruppen miteinander verknüpft sind.

Für B soll unter dem aromatischen Ring vorzugsweise eine 1,4-Phenylen-Gruppierung, unter dem heteroaromatischen Ring bevorzugt ein Pyrimidin-2,5-diyl verstanden werden. Bevorzugt werden auch die Verbindungen, in denen B für eine direkte Bindung zwischen dem Ester-O-Atom und $R^2$ steht.

Besonders bevorzugte chirale Aryloxypropionsäureester sind Verbindungen, in denen $R^1$, A und B in Formel (I) bedeuten:

$R^1$ einen geradkettigen Alkyl- oder Alkyloxy-Rest mit 7 - 16 C-Atomen;

A einen Phenylen-, Biphenylen-, Pyrimidin-phenyl-, Phenylcarbonyloxyphenyl- oder [(Phenylcarbonyloxy)-phenylcarbonyloxy]-phenylrest; und

B eine chemische Bindung oder einen Phenylen-, Biphenylen- oder Pyrimidinphenylrest.

Die erfindungsgemäßen Verbindungen der allgemeinen Formel (I) sind zweifach chiral und können beispielsweise, wie in Schema 1 aufgeführt, hergestellt werden durch Umsetzung eines mesogenen Phenols (II) mit einer chiralen Propionsäureverbindung (III) zur chiralen Aryloxyverbindung (IV), die dann durch basisch oder sauer katalysierte Verseifung in die chirale Aryloxypropionsäure (V) überführt wird; Veresterung von (V) mit der Hydroxyverbindung (VI) ergibt die erfindungsgemäße Verbindung (I).

**Schema 1**

$$\underset{\text{(II)}}{R^1\text{-A-OH}} \quad + \quad \underset{\text{(III)}}{\text{X-}\overset{*}{\text{CH}}(\text{CH}_3)\text{CO}_2\text{C}_2\text{H}_5} \longrightarrow \underset{\text{(IV)}}{R^1\text{-A-O}\overset{*}{\text{CH}}(\text{CH}_3)\text{CO}_2\text{C}_2\text{H}_5}$$

$$\text{(IV)} \longrightarrow \underset{\text{(V)}}{R^1\text{-A-O}\overset{*}{\text{CH}}(\text{CH}_3)\text{CO}_2\text{H}}$$

$$\text{(V)} \quad + \quad \underset{\text{(VI)}}{\text{HO-B-}R^2} \longrightarrow \text{(I)}$$

Die Edukte (II) bzw. (VI) sind literaturbekannt, ebenso wie das chirale Propionsäurederivat (III), bei dem X folgende Bedeutung haben kann: OH, Halogen, 4-Toluolsulfonyloxy, Methylsulfonyloxy, Trifluormethylsulfonyl.

Die Verfahren, mit denen die Verbindungen (II) und (III) racemisierungsfrei miteinander zu den Verbindungen (IV) verknüpft werden, sind ebenfalls dem Fachmann aus der Literatur bekannt.

So können Alkaliphenolate mit 2- (Sulfonyloxy)-carbonsäureverbindungen unter Erhalt der Chiralität zu Phenyloxycarbonsäureverbindungen umgesetzt werden, wie von Burkard und Effenberger, "Racemisierungsfreie Substitution von 2- (Sulfonyloxy)carbonsäureestern mit Sauerstoff- und Schwefelnucleophilen", in Chem. Ber. 119, 1594 - 1612 (1986), beschrieben.

Die Reaktion von Phenolen mit Alkoholen zu Phenolalkylethern unter Zuhilfenahme von Diethylazodicarboxylat und Triphenylphosphin, wie sie von Mitsunobu, "The Use of Diethyl Azodicarboxylate and Triphenylphosphine in Synthesis and Transformation of Natural Products", in Synthesis 1981, 1 - 28, beschrieben wird, kann auch zur Herstellung von (IV) und somit letztlich von (I) verwendet werden, wenn ein Milchsäureethylester (III, X = OH) mit einem mesogenen Phenol (II) zur Reaktion gebracht wird.

Die Verseifung von (IV) zu (V) und Veresterung von (V) mit (VI) zu (I) können nach dem Fachmann bekannten Methoden erfolgen.

Gegenstand der Erfindung sind auch verdrillbare Flüssigkristall-Phasen mit einem Gehalt an mindestens einer chiralen Verbindung, die als chirale Verbindung mindestens eine Verbindung der allgemeinen Formel (I) enthalten. Unter dem Begriff "verdrillbare Flüssigkristall-Phase" sind nematische, cholesterische oder geneigt("tilted")-smektische, insbesondere SmC-Phasen zu verstehen.

Die verdrillbaren Flüssigkristall-Phasen bestehen aus 2 bis 20, vorzugsweise 2 bis 15 Komponenten, darunter mindestens einer der erfindungsgemäß beanspruchten zweifach chiralen Verbindungen. Die anderen Bestandteile werden vorzugweise ausgewählt aus den bekannten Verbindungen mit nematischen, cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Phasen bereits vor der Zugabe der chiralen Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristall-Phase zeigt, d.h., mindestens eine enantiotrope (Klärtemperatur > Schmelztemperatur) oder monotrope (Klärtemperatur < Schmelztemperatur) Mesophasenbildung erwarten läßt.

Insbesondere enthält die verdrillbare Flüssigkristall-Phase neben mindestens einer der erfindungsgemäß beanspruchten zweifach chiralen Verbindungen eine Ester-Verbindung mit $S_c$-Phase, z.B. 4-Alkyloxybenzoesäure-phenylester, oder eine Phenylpyrimidinverbindung mit $S_c$-Phase, z.B. ein 4- (5-Alkyl-pyrimidin-2-yl)-1-alkoxy-benzol. Von der oder den erfindungsgemäßen Verbindung(en) enthalten die Flüssigkristall-Phasen im allgemeinen 0,01 bis 70 Gew.-%, insbesondere 0,05 bis 50 Gew.-%.

Die erfindungsgemäßen Verbindungen sind insbesondere als Dotierstoffe für geneigt-smektische Flüssigkristall-Phasen geeignet, da sie diese in ferroelektrische Flüssigkristall-Phasen umwandeln; die Werte für die spontane Polarisation (Ps) liegen im Bereich von etwa 10 bis 120 $nC \cdot cm^{-2}$ (linear extrapoliert auf die reine Verbindung).

**Herstellungsbeispiele**

A (R)-(+)-2-[4-(5-Octyl-pyrimidin-2-yl)phenyl]oxypropionsäureoctylester

(I, $R^1$:$C_8H_{17}$, A: 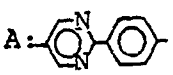 B: -, $R^2$:$C_8H_{17}$)

In 75 ml Tetrahydrofuran werden 5,25 g (0,02 mmol)
Triphenylphosphin und 3,15 ml (0,02 mol)
Azodicarbonsäurediethylester bei 0°C gelöst. Nach 15 min werden 5,69 g (0,02 mol) 4- (5-Octyl-pyrimidin-2-yl)phenol

(II, $R^1$:$C_8H_{17}$, A: 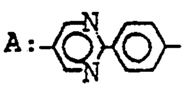 )

und 2,36 g (0,02 mol) (S)-(-)-Milchsäureethylester (III, X:OH) zugegeben. 16 h später wird im Vakuum zur Trockne gebracht und der Rückstand über 200 g Kieselgel mit Dichlormethan als Eluens chromatographiert. Es resultieren 5,7 g (74%) eines farblosen Öles

(IV, $R^1$:$C_8H_{17}$, A: 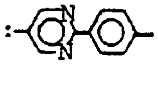 ).

Es wird in 30 ml Methanol aufgenommen und bei 15°C tropfenweise mit 33 %iger Natronlauge versetzt, bis bei Lauge-Zugabe keine Ausfällung mehr auftritt und sich der Ester (IV) dünnschicht-chromatographisch nicht mehr nachweisen läßt. Durch Zugabe von 10 n HCl wird auf pH 2 gestellt und die ausgefallene Säure

(V, $R^1$:$C_8H_{17}$, A: 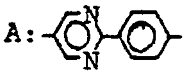 )

mit Wasser gewaschen und getrocknet:
4,9 g (93 %) farblose Kristalle vom Schmp. 101 - 102°C;

$[\alpha]_D^{22}$ + 19,6 (c=5, $CH_2Cl_2$).

1,5 g (0,004 mol) dieser Carbonsäure wird mit 0,65 g (0,005 mol) n-Octanol (VI, B: -, $R^2$:$C_8H_{17}$), 1,03 g (0,005 mol) Dicyclohexylcarbodiimid und 0,005 g 4-Dimethylaminopyridin in 30 ml Dichlormethan vereinigt.

Nach 24stündigem Stehen wird filtriert, das Filtrat über Kieselgel mit Dichlormethan chromatographiert; es resultieren 1,27 g (68 %) eines farblosen Öles.

$[\alpha]_D^{22}$: +31,6 (c=5, $CHCl_3$)

$^1$H-NMR und IR-Spektrum sowie die Elementaranalyse stützen die angegebene Struktur.

B (S)-(-)-2-[4-(4-Decyloxyphenylcarbonyloxy)phenyl]-oxypropionsäureethylester

(I, $R^1$:$C_{10}H_{21}O$, A: 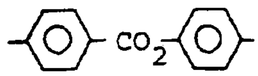 , B: -, $R^2$:$C_2H_5$)·

10 g (0,033 mol) (S)-(-)-2-[4-Benzyloxy-phenyl]-oxypropionsäureethylester

(IV, $R^1$:$C_6H_5CH_2O$-, A: 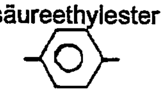 ),

analog obiger Vorschrift aus 4-Benzyloxy-phenol

(I, $R^1$:$C_6H_5CH_2O$, A: 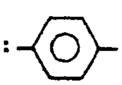 )

und (R)-(1)-Milchsäureethylester (III, X:OH) mit der Mitsonobu-Reaktion erhalten, werden in 100 ml Tetrahydrofuran mit 1 g Pd/C (10 %) bei 27°C und Normaldruck bis zum Ende der $H_2$-Aufnahme hydriert. Der Katalysator wird abfiltriert, das Lösungsmittel abdestilliert, es resultieren 6,5 g (S)-(-)-2-(4-Hydroxyphenyl)oxypropionsäureethylester

(IV, $R^1$: -, A:HO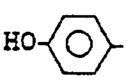 , B: -, $R^2$:$C_2H_5$)

$[\alpha]^{25}$: -43,8°(neat). Eine Lösung von 6,1 g (0,029 mol) dieser Verbindung in 20 ml Pyridin wird bei 10°C tropfenweise mit 8,9 g (0,03 mol) 4-Decyloxy-benzoylchlorid versetzt. Nach 3stündigem Rühren bei 10°C wird in 200 ml Eiswasser gegossen, durch Zugabe von HCl pH 3 eingestellt und mit Dichlormethan extrahiert. Der Extrakt wird an Kieselgel mit Dichlormethan chromatographiert und die produkthaltige Fraktion aus Methanol umkristallisiert: 4,1 g (40 % d.Th.) farblose Kristalle; $[\alpha]_D^{23}$: -26,1° (c=1, $CHCl_3$).

$^1$H-NMR- und IR-Spektrum sowie die Elementaranalyse sind in Einklang mit der angegebenen Struktur.

Bei Einsatz der entsprechenden Edukte sind diese Vorschriften zur Herstellung der Verbindungstypen A und B aus dem Stand der Technik repräsentativ für alle erfindungsgemäßen Verbindungen der nachfolgenden Tabelle 1.

Tabelle I

$R^1-A-O-\overset{CH_3}{C^{*}H}-CO_2-B-R^2$

| Nr. | $R^1$ | –A– | –B– | –$R^2$ | Phasenfolge (°C) K | $S_A$ | $N^*$ | I |
|---|---|---|---|---|---|---|---|---|
| 1 | $C_8H_{17}$ | 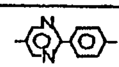 | -- | (S)-$C^{*}H_2CH(Cl)CH_3$ | ·32 | -- | -- | · |
| 2 | " | " | -- | (S)-$C^{*}H(CH_3)C_2H_5$ | ·-50 | -- | -- | · |
| 3 | " | " | -- | (S)-$CH_2C^{*}H(CH_3)C_2H_5$ | ·-50 | -- | -- | · |
| 4 | $C_8H_{17}$ | 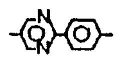 | -- | (2S,3S)-$CH_2-\overset{*}{CH}-\overset{*}{CH}-C_3H_7$ (O bridging the two CH) | ·33 | -- | -- | · |
| 5 | " | " | 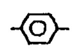 | (R)-$OC^{*}H(CH_3)CO_2C_2H_5$ | ·51 | -- | -- | · |
| 6 | " | " | " | (S)-$OC^{*}H(CH_3)CO_2C_2H_5$ | ·69 | -- | -- | · |
| 7 | $C_{10}H_{21}O$ | 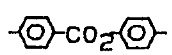 | -- | (S)-$CH_2C^{*}H(CH_3)C_2H_5$ | · 9 | -- | -- | · |

Tabelle I (Fortsetzung)

$$R^1\text{-}A\text{-}O\text{-}\overset{CH_3}{\underset{*}{C}}H\text{-}CO_2\text{-}B\text{-}R^2$$

| Nr. | $R^1$ | -A- | -B- | $-R^2$ | Phasenfolge (°C) K | $S_A$ | $N^*$ | I |
|---|---|---|---|---|---|---|---|---|
| 8 | $C_{16}H_{33}O$ | " | -- | " | ·44 | -- | -- | · |
| 9 | $C_8H_{17}O$ | 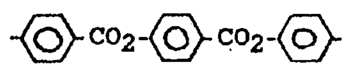 | -- | (S)-$CH_2\underset{*}{C}H(CH_3)C_2H_5$ | ·84 | ·93 | ·95 | · |
| 10 | " | 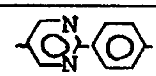 | -- | (S,S)-$CH_2\underset{*}{C}H(Cl)$-$\underset{*}{C}H(CH_3)$-$C_2H_5$ | 25 | - | - | · |

Die Verbindungen 1 - 6 haben R-, die Verbindungen 7 - 10 S-Konfiguration am zentralen asymmetrischen C-Atom.

**Anwendungsbeispiele 1 bis 10**

Zur Uberprüfung der Wirksamkeit der vorstehend beschriebenen Verbindungen als Dotierstoffe in Flüssigkristall-Systemen werden diese in Konzentrationen von jeweils 10 Mol-% mit den Racematen der Verbindungen A oder B oder dem Enantiomer C

A 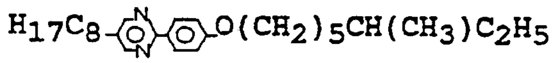

B 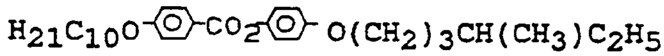

C 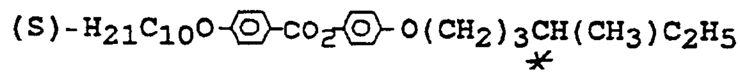

gemischt und jeweils die Werte für die spontane Polarisation ($P_5$ in $nC \cdot cm^{-2}$), für die Schaltzeit $\tau$ (in µs bei ± $10^7$ $Vm^{-1}$) und für den optischen Neigungswinkel der SmC*-Phase θ (in °) der Mischung bestimmt. Die $P_5$-Werte werden nach der Methode von Sawyer et al. (Phys. Rev. 35, 269 bis 273, 1930) gemessen, wobei eine spezielle Meßzelle [Skarp et al. in Ferroelectric Letters Vol. 06, 000 (1986)] verwendet wird, in der auch die τ- und θ-Werte bestimmt werden. Bei einer Zellenschichtdicke von 2 µm wird durch Scherung eine einheitliche planare Orientierung der Flüssigkristalle in der SmC*-Phase erreicht [SSFLC-Technik, Clark et al., Appl. Phys. Lett. 36, 899 (1980)]. Nach Anordnung der Meßzelle zwischen zwei gekreuzte Polarisatoren erfolgt die Bestimmung der Schaltzeit mit Hilfe einer Photodiode und die des optischen Neigungswinkels bzw. des Schaltwinkels durch Drehung der Meßzelle von maximalem zu minimalem Lichtdurchgang durch die Meßanordnung. Tabelle II faßt die Ergebnisse für die Mischungen zusammen. Spalte 1 gibt die Nr. der erfindungsgemäßen Verbindung gemäß Tabelle I an, Spalte 2 die Mischungskomponente A, B oder C, Spalte 3 den Smc*-Bereich, wobei die Werte in Klammern die unterkühlbare Grenze des SmC*-Bereichs angeben, Spalte 4 die spontane Polarisation, Spalte 5 die Schaltzeit, Spalte 6 den Schaltwinkel und Spalte 7 die Meßtemperatur.

Tabelle II

| Verb. aus Tabelle I | Mischung mit | $S_C^*$-Bereich (°C) | $P_S$ ($nC \cdot cm^{-2}$) | τ (µs) | 2 θ (grad) | Temp. (°C) |
|---|---|---|---|---|---|---|
| 1 | A | 11[5]-30 | < 5 | < 3 | 2 | 25 |
| 2 | A | 10[0]-18 | - 10 | 22 | 9 | 5 |
| 3 | A | 11[-3]24 | - 10 | < 2 | 25 | 20 |
| 4 | A | 13[1]-23 | -- | -- | -- | -- |
| 5 | A | 13[5]-42 | + 22 | 280 | 33 | 25 |
| 6 | A | 13[10]-41 | + 14 | 350 | 40 | 25 |
| 7 | B | 25[24]-55 | - 107 | 40 | 46 | 40 |
| 8 | B | 30[30]-58 | - 115 | 57 | 57 | 40 |
| 9 | B | 33[32]-58 | - (< 5) | 130 | 37 | 40 |
| 10 | B | 25[23]-50 | -110 | 53 | 50 | 40 |

## Patentansprüche

1. Chirale Aryloxypropionsäureester der allgemeinen Formel (I)

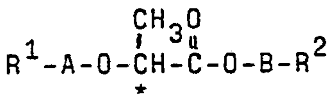 (I)

in der $R^1$, A, B und $R^2$ bedeuten:

$R^1$ einen geradkettigen Alkyl- oder Alkyloxyrest mit 1-16 C-Atomen oder einen verzweigten Alkyl- oder Alkyloxyrest mit 4-16 C-Atomen, wobei eine oder zwei nicht-benachbarte $CH_2$-Gruppen durch ein S und-/oder O ersetzt sein können;

A einen, zwei oder drei aromatische oder heteroaromatische Ringe, die direkt oder durch eine bzw. zwei COO-Gruppe(n) miteinander verknüpft sind;

B eine chemische Bindung oder einen oder zwei aromatische oder heteroaromatische Ringe, die direkt miteinander verknüpft sind;

$R^2$ a) wenn B eine cehmische Bindung ist, einen Alkylrest mit 2-10 C-Atomen, der ein asymmetrisches C-Atom enthält, das mit $CH_3$, Halogen oder einer $COOC_2H_5$-Gruppe substituiert ist, oder einen Alkylrest mit 3-10 C-Atomen, der zwei benachbarte asymmetrische C-Atome enthält, von denen eines mit Halogen und das andere mit einer $CH_3$-Gruppe substituiert ist, oder die gemeinsam mit einem O einen Oxiranring bilden; oder

b) wenn B einen oder zwei aromatische oder heteroaromatische Ringe bedeutet, die direkt miteinander verknüpft sind, einen Alkyloxyrest mit derselben Anzahl von C-Atomen und demselben Aufbau der Alkylgruppe wie unter a).

2. Chirale Aryloxypropionsäureester nach Anspruch 1, dadurch gekennzeichnet, daß in der allgemeinen Formel (I) bedeuten:

$R^1$ einen geradkettigen Alkyl- oder Alkyloxyrest mit 7-16 C-Atomen;

A einen Phenylen-, Biphenylen-, Pyrimidyl-phenyl-, Phenyl-carbonyloxyphenyl- oder [(Phenylcarbonyloxy)-phenylcarbonyloxy]-phenylrest; und

B eine chemische Bindung oder einen Phenylen-, Biphenylen-oder Pyrimidinphenylrest.

3. Verdrillbare Flüssigkristall-Phase mit einem Gehalt an mindestens einer chiralen Verbindung der allgemeinen Formel (I) nach Anspruch 1.

4. Verdrillbare Flüssigkristall-Phase mit einem Gehalt an mindestens einer chiralen Verbindung der allgemeinen Formel (I) nach Anspruch 2.

5. Verdrillbare Flüssigkristall-Phase nach Anspruch 3 oder 4, dadurch gekennzeichnet, daß sie neben der Verbindung der allgemeinen Formel (I) eine Ester-Verbindung mit $S_c$-Phase enthält.

6. Verdrillbare Flüssigkristall-Phase nach Anspruch 5, dadurch gekennzeichnet, daß sie als Ester-Verbindung mit $S_c$-Phase einen 4-Alkoxybenzoesäure-phenylester enthält.

7. Verdrillbare Flüssigkristall-Phase nach Anspruch 3 oder 4, dadurch gekennzeichnet, daß sie neben der Verbindung der allgemeinen Formel (I) eine Phenylpyrimidinverbindung mit $S_c$-Phase enthält.

8. Verdrillbare Flüssigkristall-Phase nach Anspruch 7, dadurch gekennzeichnet, daß sie als Phenylpyrimidinverbindung mit $S_c$-Phase 4-(5-Alkylpyrimidin-2-yl)-1-alkoxybenzol enthält.

9. Anzeigeelement enthaltend eine Flüssigkristall-Phase nach einem der Ansprüche 3 bis 8.

10. Verfahren zur Umwandlung einer geneigt-smektischen in eine ferroelektrische Flüssigkristall-Phase durch Zusatz mindestens einer Verbindung der allgemeinen Formel (I) nach Anspruch 1 oder 2.

## Claims

1. A chiral aryloxypropionate of the formula (I)

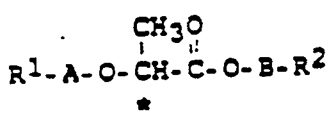 (I)

in which $R^1$, A, B and $R^2$ have the following meanings:

$R^1$ denotes a straight-chain alkyl or alkoxy radical having 1 to 16 carbon atoms or a branched alkyl or alkoxy radical having 4 to 16 carbon atoms, it being possible for one or two non-adjacent $CH_2$ groups to be replaced by a sulfur and/or oxygen atom;

A denotes one, two or three aromatic or heteroaromatic rings which are linked to one another directly or via one or two COO group(s);

B denotes a chemical bond or one or two aromatic or heteroaromatic rings which are linked directly to one another;

$R^2$ a) if B is a chemical bond, denotes an alkyl radical having 2 to 10 carbon atoms which contains an asymmetrical carbon atom which is sub-stituted by $CH_3$, halogen or a $COOC_2H_5$ group, or an alkyl radical having 3 to 10 carbon atoms which contains two adjacent, asymmetrical carbon atoms of which one is substituted by halogen and the other by a $CH_3$ group, or which, together with an 0, form an oxirane ring, or

b) if B denotes one or two aromatic or heteroaromatic rings which are linked directly to one another, denotes alkoxy radicals having the same number of carbon atoms and the same structure of the alkyl group as under a).

2. A chiral aryloxypropionate as claimed in claim 1, wherein $R^1$, A, B and $R^2$ in the general formula (I) have the following meaning:

$R^1$ denotes a straight-chain alkyl or alkoxy radical having 7 - 16 carbon atoms,

A denotes a phenylene, biphenylene, pyrimidylphenyl, phenylcarbonyloxyphenyl or [(phenylcarbonyloxy)-phenylcarbonyloxy]phenyl radical, and

B denotes a chemical bond or a phenylene, biphenylene or pyrimidinephenyl radical.

3. A twistable-liquid crystal phase containing at least one chiral compound of the general formula (I) as claimed in claim 1.

4. A twistable liquid-crystal phase containing at least one chiral compound of the general formula (1) as claimed in claim 2.

5. A twistable liquid-crystal phase as claimed in claim 3 or 4, which contains, besides the compound of the general formula (I), an ester compound having an $S_c$ phase.

6. A twistable liquid-crystal phase as claimed in claim 5, which contains, as the ester compound having an $S_c$ phase, a phenyl 4-alkoxybenzoate.

7. A twistable liquid-crystal phase as claimed in claim 3 or 4, which contains, besides the compound of the general formula (I), a phenylpyrimidine compound having an $S_c$ phase.

8. A twistable liquid-crystal phase as claimed in claim 7, which contains, as the phenylpyrimidine compound having an $S_c$ phase, 4-(5-alkylpyrimidine-2-yl)-1-alkoxybenzene.

9. A display element containing a liquid-crystal phase as claimed in any of claims 3 to 8.

10. A process for converting a tilted smectic liquid-crystal phase into a ferroelectric liquid-crystal phase by adding at least one compound of the general formula (I) as claimed in claim 1 or 2.

## Revendications

1. Esters d'acides aryloxypropioniques chiraux, de formule générale (I) :

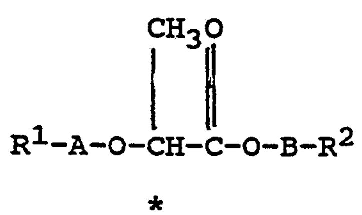

(I)

dans laquelle les symboles $R^1$, A, B et $R^2$ ont les sens suivants :

$R^1$ représente un reste alkyle ou alkyloxy linéaire ayant 1 à 16 atomes de carbone ou un reste alkyle ou alkyloxy ramifié ayant 4 à 16 atomes de carbone, un ou deux groupes $CH_2$ non voisins pouvant être remplacés par S et/ou O ;

A représente un, deux ou trois noyaux aromatiques ou hétéro-aromatiques, qui sont reliés l'un à l'autre directement ou par l'intermédiaire d'un ou deux groupes COO-;

B représente une liaison chimique ou un deux noyaux aromatiques ou hétéro-aromatiques, qui sont reliés

directement l'un à l'autre;

$R^2$ représente :

a) lorsque B est une liaison chimique, $R^2$ représente un reste alkyle ayant 2 à 10 atomes de carbone, qui contient un atome de C asymétrique portant comme substituant un groupe $CH_3$, halogéno ou $COO_2H_5$, ou bien

$R^2$ représente un reste alkyle ayant 3 à 10 atomes de carbone, qui contient 2 atomes de C asymétriques voisins, dont l'un porte un halogène comme substituant et dont l'autre porte un groupe $CH_3$ comme substituant, ou bien ces deux atomes de carbone forment avec un O un noyau oxiranne; ou bien

b) lorsque B représente un ou deux noyaux aromatiques ou hétéro-aromatiques, qui sont directement reliés l'un à l'autre, $R^2$ représente un reste alkyloxy comportant le même nombre d'atomes de carbone et ayant la même structure de groupe alkyle que ce qui est décrit en a).

2. Esters d'acides aryloxypropioniques chiraux selon la revendication 1, caractérisés en ce que, dans la formule générale (I) :

$R^1$ représente un reste alkyle ou alkyloxy linéaire comportant 7 à 16 atomes de carbone;

A représente un reste phénylène, biphénylène, pyrimidyle-phényle, phényl-carbonyloxyphényle ou [(phénylcarbonyloxy)-phénylcarbonyloxy)]-phényle et

B représente une liaison chimique ou un reste phénylène, biphénylène, ou pyrimidyl-phényle.

3. Phase de cristaux liquides pouvant être torsadés, comportant au moins un composé chiral de formule générale (I) selon la revendication 1.

4. Phase de cristaux liquides pouvant être torsadés et contenant au moins un composé chiral de formule générale (I) selon la revendication 2.

5. Phase de cristaux liquides pouvant être torsadés selon la revendication 3 ou 4, caractérisée en ce qu'elle contient, en plus du composé de formule générale (I), un ester présentant une phase $S_c$.

6. Phase de cristaux liquides pouvant être torsadés selon la revendication 5, caractérisée en ce qu'elle contient, comme ester comportant une phase $S_c$, un ester phénylique d'acide 4-alcoxybenzoïque.

7. Phase de cristaux liquides pouvant être torsadés selon la revendication 3 ou 4, caractérisée en ce qu'elle contient, en plus du composé de formule générale (I), un composé phénylpyrimidinique comportant une phase $S_c$.

8. Phase de cristaux liquides pouvant être torsadés selon la revendication 7, caractérisée en ce qu'elle contient comme composé phénylpyrimidinique à phase $S_c$ un 4-(5-alkylpyrimidine-2-yl)-alcoxybenzène.

9. Eléments d'affichage contenant une phase de cristaux liquides selon une des revendications 3 et 8.

10. Procédé pour transformer une phase smectique inclinée de cristaux liquides en une phase ferro-électrique de cristaux liquides, par addition d'au moins un composé de formule générale (I) selon la revendication 1 ou 2.